# EUROPÄISCHE PATENTSCHRIFT

(11) **EP 2 709 702 B1**
(45) Veröffentlichungstag und Bekanntmachung des Hinweises auf die Patenterteilung: **25.05.2016**
(21) Anmeldenummer: 12727613.7
(22) Anmeldetag: 18.05.2012
(51) Int. Cl.: A61M 15/06, A61M 15/00

(54) **INHALATIONS-PEN**
INHALATION PEN
CRAYON D'INHALATION

(30) Priorität: 20.05.2011 DE 202011101012 U
(43) Veröffentlichungstag der Anmeldung: 26.03.2014
(73) Patentinhaber: Haindl, Hans, 30974 Wennigsen (DE)
(72) Erfinder: Haindl, Hans, 30974 Wennigsen (DE)
(74) Vertreter: Vossius & Partner Patentanwälte Rechtsanwälte mbB
(86) Internationale Anmeldenummer: PCT/EP2012/059283
(87) Internationale Veröffentlichungsnummer: WO 2012/159996

(56) Entgegenhaltungen:
- EP-A1- 1 522 325
- WO-A1-00/09188
- WO-A1-01/26720
- WO-A1-03/103760
- DE-U1- 8 801 258
- GB-A- 2 266 222
- KR-B1- 101 011 453
- RU-C2- 2 158 573
- US-A- 5 894 841

## Beschreibung

Die vorliegende Erfindung betrifft einen Inhalator zur Verabreichung einer definierten Dosis eines Wirkstoffes, insbesondere eines Medikamentes, in die Atemwege eines Anwenders.

Für die Behandlung verschiedenster Atemwegserkrankungen, aber teilweise auch zur Aufnahme von Medikamenten zu anderen Zwecken, wird eine Fülle von Inhalationsgeräten verwendet. Zu nennen sind hier die sogenannten Metered Dose Inhaler (MDI), die aus einer durch ein Treibmittel unter Druck gesetzten Kartusche auf Druck eine definierte Menge eines flüssigen Medikamentes abgeben, sowie die sogenannten Pulverinhalatoren, bei denen sich eine Dosis Pulver in einer Gelatine- oder Kunststoffkapsel befindet, im Inhalator geöffnet wird und das Pulver durch den Atemluftstrom des Anwenders aufgewirbelt und fein verteilt eingeatmet wird. Andere Inhalatoren basieren z.B. auf dem Zerstäuben eines Medikamentes in Mikrodüsen.

All diese Systeme funktionieren mehr oder weniger gut, haben jedoch in unterschiedlichem Maße den Nachteil, dass es sich um relativ sperrige Geräte handelt, die z. B. in leichter Sommerkleidung oder beim Sport nicht unbedingt unauffällig am Körper zu tragen sind. Da die entsprechenden Patienten in der Regel jedoch darauf angewiesen sind, die nötigen Medikamente jederzeit bei sich zu haben, ist es wünschenswert, einen Inhalator bereitzustellen, der derart ausgebildet ist, dass er ohne Beeinträchtigung am Körper zu tragen ist.

Folgendes sind relevanten Stand der Technik: EP1522325 A1, WO 01/26720 A1, WO 03/103760 A1, RU 2158573 C2, US 5894841 A. Die Erfindung ist durch die beigefügten Ansprüche definiert. Die vorliegende Erfindung basiert unter anderem auf der Idee, einen voll funktionsfähigen Inhalator, der bevorzugt mehrere Medikamenteneinzeldosen und/oder eine Tagesdosis beinhaltet, in der Form eines Kugelschreibers auszubilden. Mit anderen Worten soll der erfindungsgemäße Inhalator eine längliche, relativ schlanke Form aufweisen, die es bevorzugt erlaubt, den Inhaltor z.B. in einer Hemd- oder Jackentasche bei sich zu tragen. Hierfür kann der Inhalator bevorzugt einen Clip aufweisen, der dazu Erscheinung einem Kugelschreiber und ist auf den ersten Blick von einem solchen optisch nicht zu unterscheiden. So kann der Inhalator beispielsweise einen Druckknopf zum Auslösen einer Medikamentendosis aufweisen, der den üblichen Kugelschreiberknöpfen gleicht.

Die vorliegende Erfindung betrifft demnach einen Inhalator zur, bevorzugt wiederholten, Verabreichung einer definierten Dosis eines Wirkstoffes in die Atemwege eines Anwenders. Der Inhalator hat eine längliche Form, die eine Längsache und ein Querschnittsprofil definiert, wobei die maximale Querschnittsfläche höchstens 4 cm² und die maximale lineare Ausdehnung des Querschnitts höchstens 2,85 cm beträgt.

Das Querschnittsprofil an unterschiedlichen axialen Positionen wird dabei jeweils durch einen Schnitt senkrecht zur Längsachse definiert. Die maximale Querschnittsfläche ergibt sich aus dem Maximum der so definierten Querschnittsflächen an sämtlichen axialen Positionen. Gleichermaßen lässt sich in jedem Querschnitt eine maximale Ausdehnung bestimmen, z.B. der Durchmesser im Falle eines Kreises oder die Diagonale im Falle eines Rechtecks. Die maximale Ausdehnung sämtlicher Querschnitte senkrecht zur Längsachse soll demnach höchstens 2,85 cm betragen. Mit anderen Worten soll der größte Querschnitt im Extremfall nicht stärker von einem Kreis abweichen als ein Quadrat. Der Querschnitt soll sich im Regelfall auf denjenigen der länglichen Form als solcher beziehen und etwaige zusätzlich angebrachte Komponenten, wie z.B. einen Clip zum Befestigen, nicht mitumfassen.

Gemäß einer bevorzugten Ausführungsform des erfindungsgemäßen Inhalators beträgt die maximale Querschnittsfläche höchstens 3 cm², bevorzugt höchstens 2 cm², besonders bevorzugt höchstens 1,5 cm². Gemäß einer bevorzugten Ausführungsform des erfindungsgemäßen Inhalators beträgt die maximale Ausdehung des Querschnitts höchstens 2,2 cm, bevorzugt höchstens 1,5 cm, besonders bevorzugt höchstens 1,1 cm.

Gemäß einer bevorzugten Ausführungsform des erfindungsgemäßen Inhalators weist dieser im wesentlichen die Form eines Kugelschreibers auf. Der Querschnitt des Inhalator kann beispielsweise kreisförmig, elliptisch, quadratisch, rechteckig, fünfeckig, sechseckig Inhalators beträgt bevorzugt zwischen 8 cm und 18 cm, stärker bevorzugt zwischen 10 cm und 16 cm, besonders bevorzugt zwischen 12 cm und 15 cm.

Gemäß einer bevorzugten Ausführungsform des erfindungsgemäßen Inhalators weist dieser einen Clip auf, der bevorzugt dazu geeignet ist, den Inhalator in einer Hemd- und/oder Jackentasche zu fixieren.

Gemäß einer bevorzugten Ausführungsform weist der Inhalator an einem ersten Ende eine Ansaugöffnung auf. Diese hat bevorzugt eine Öffnungsfläche zwischen 10 mm² und 40 mm², besonders bevorzugt zwischen 15 mm² und 25 mm². Bevorzugt ist die Ansaugöffnung im wesentlichen kreisrund mit einem Öffnungsdurchmesser zwischen 3 mm und 7 mm, besonders bevorzugt zwischen 4 mm und 6 mm.

Bevorzugt ist das erste Ende derart beweglich, dass es sich aus einer ersten Position, in der das erste Ende im wesentlichen parallel zur Längsachse des Inhalators ausgerichtet ist, in eine zweite Position bewegen lässt, in der das erste Ende mit der Längsachse des Inhalators einen Winkel von mindestens 45°, bevorzugt von mindestens 60°, stärker bevorzugt von mindestens 75°, besonders bevorzugt von etwa 90°, einschließt. Dabei ist das erste Ende gegenüber der Längsachse des Inhalators bevorzugt verkippbar und/oder verdrehbar. Alternativ oder zusätzlich kann das erste Ende hierfür flexibel bzw. verbiegbar sein. Dadurch kann der Inhalator unabhängig davon, ob er vom Anwender aufrecht stehend oder sitzend oder aber im Liegen benutzt wird, im wesentlichen etwa senkrecht ausgerichtet bleiben.

Der Wirkstoff kann grundsätzlich in Form einer Flüssigkeit, bevorzugt einer volatilen Flüssigkeit, oder in Form eines Pulvers vorliegen. Der Wirkstoff enthält bevorzugt eines oder mehrere Medikamente.

Gemäß einer bevorzugten Ausführungsform weist der Inhalator eine Mischeinrichtung auf, die dazu geeignet ist, einen Luftstrom innerhalb des Inhalators zu verwirbeln. Der Luftstrom entsteht bevorzugt, indem der Anwender Luft durch die Ansaugöffnung ansaugt. Der Inhalator weist hierfür bevorzugt eine oder mehrere Öffnungen zum Gemäß einer bevorzugten Ausführungsform weist der Inhalator ein Sichtfenster auf, durch das die Anzahl der vorhandenen Dosen optisch überprüft werden kann. Dies kann derart angeordnet und dimensioniert sein, dass erkennbar ist, ob sich noch eine vorbestimmte Mindestanzahl von Dosen (z.B. noch mindestens drei, mindestens fünf oder mindestens zehn Dosen) im Inhalator befindet.

Gemäß einer bevorzugten Ausführungsform des erfindungsgemäßen Inhalators weist dieser einen Auslösemechanismus auf, wobei ein Betätigen des Auslösemechanismus bewirkt, dass die definierte Dosis des Wirkstoffes bereitgestellt wird. Das Bereitstellen kann je nach Art des Inhalators bzw. des Wirkstoffes ein Abmessen und/oder Ausgeben einer definierten Flüssigkeitsmenge oder das Fördern und/oder Ausgeben einer bereits zuvor definierten Dosis eine Flüssigkeit oder eines Pulvers umfassen.

Bevorzugt weist der Auslösemechanismus einen Druckknopf auf, der bevorzugt an dem dem ersten Ende gegenüberliegenden Ende des Inhalators angebracht ist. Vorzugsweise ist der Druckknopf derart ausgebildet, dass er im Aussehen dem Druckknopf üblicher Kugelschreiber entspricht. Ein einmaliges Betätigen des Druckknopfes bewirkt dann die Freigabe bzw. Bereitstellung einer Wirkstoffdosis.

Gemäß einer bevorzugten Ausführungsform des erfindungsgemäßen Inhalators enthält dieser mehrere, voneinander separierte Portionen der definierten Dosis des Wirkstoffes. Die Portionen können einzeln verpackt bzw. verschlossen sein. Z.B. kann ein flüssiger Wirkstoff in einzelnen Kapseln bereitgestellt werden. Dabei bewirkt ein Betätigen des Auslösemechanismus bevorzugt, dass eine Portion der definierten Dosis des Wirkstoffes an eine vorbestimmte Position gefördert wird und/oder geöffnet wird.

Gemäß einer bevorzugten Ausführungsform des erfindungsgemäßen Inhalators sind die Portionen in Dosisbehältern auf einem Förderband, bevorzugt in Blasen bzw. Blistern eines Blisterstreifens, angeordnet. Dabei bewirkt ein Betätigen des Auslösemechanismus bevorzugt, dass das Förderband bzw. der Blisterstreifen um einen Abstand zwischen Inhalator bevorzugt eine Kanülenspitze, einen Dorn, ein Messer oder dergleichen auf. Ein Betätigen des Auslösemechanismus bewirkt bevorzugt, dass ein Dosisbehälter des Förderbands bzw. eine Blase des Blisterstreifens derart in die Kanülenspitze, den Dorn oder das Messer bewegt wird, dass dieser bzw. diese aufgeschnitten bzw. punktiert wird. Auf diese Weise wird der im Dosisbehälter bzw. in der Blase befindliche Wirkstoff freigegeben und kann durch ein Ansaugen von Luft in die Atemwege des Anwenders aufgenommen werden. Im Falle eines pulverförmigen Wirkstoffes wird dabei die Blase bevorzugt derart aufgeschnitten, dass der Wirkstoff aus der Blase herausfallen bzw. - rieseln kann. Enthält die Blase einen flüssigen Wirkstoff, so ist dieser bevorzugt volatil, so dass ein Öffnen oder Punktieren ein rasches Verdunsten bewirkt. Der verdunstete Wirkstoff kann dann beim Ansaugen einfach eingeatmet werden.

Gemäß einer bevorzugten Ausführungsform des erfindungsgemäßen Inhalators greift der Auslösemechanismus, d.h. beispielsweise der Druckknopf, mittels eines Hakens oder mittels Zähnen in sperrklinkenartige Vorsprünge an oder Öffnungen in dem Förderband bzw. Blisterstreifen ein. Auf diese Weise fördert der Auslösemechanismus das Förderband um eine Position weiter und fördert derart z.B. die nächste gefüllte Blase an oder in das Messer oder den Dorn. Anschließend wird der Haken zurückgezogen, um in den nächsten Vorsprung eingreifen zu können. Dies geschieht beispielsweise durch Drücken und Loslassen eines gefederten Druckknopfes. Der Auslösemechanismus kann auch durch Drehen eines, vorzugsweise federgespannten, Drehknopfes oder dergleichen erfolgen.

Gemäß einer bevorzugten Ausführungsform des erfindungsgemäßen Inhalators weist dieser eine flexible Auflagefläche auf, die derart angeordnet ist, dass eine Portion der definierten Dosis des Wirkstoffes auf ihr gelagert bzw. gehalten werden kann. So fällt bzw. rieselt beispielsweise eine Pulverportion aus einer aufgeschnittenen Blase auf diese Auflagefläche, wenn der Auslösemechanismus betätigt wird. Ein anschließendes Saugen an der Ansaugöffnung bewirkt dann bevorzugt, dass die flexible Auflagefläche soweit deformiert wird, dass die darauf gelagerte Portion freigegeben wird. Auf diese Weise wird verhindert, dass beim Aufschneiden der Blase der Wirkstoff aus der Ansaugöffnung herausfällt, bevor an dieser ordnungsgemäß gesaugt wirkt.
werden. Hierfür wird bevorzugt die Form der Druckkartuschen derart geändert, dass diese länger und dünner als herkömmliche Druckkartuschen sind. Ferner kann das erfindungsgemäße Prinzip auch auf Pulverinhalatoren angewendet werden, bei denen die definierte Wirkstoffdosis in Form einer definierten Pulvermenge mittels eines Schabemechanismus von einem Wirkstoffblock abgeschabt und dadurch bereitgestellt und verabreicht wird.

Der erfindungsgemäße Inhalator ist unter anderem vorteilhaft, da er einerseits ausgesprochen klein und andererseits derart ausgeformt ist, dass er sich ohne Beeinträchtigung permanent am Körper tragen lässt, beispielsweise in einer Hemd- oder Jackentasche. Auf diese Weise muss der Anwender keinen Inhalator einer womöglich unhandlichen Form und Größe in hinreichend großen Taschen mit sich herumtragen. Zum anderen lässt sich mit Hilfe der kugelschreiberartigen Form leicht verbergen, dass der Anwender auf eine permanente Medikation angewiesen ist. Trotz der kleinen und simplen Form des erfindungsgemäßen Inhalators ermöglicht dieser das Mitführen der Inhalationsdosen für einen Tag, bevorzugt für eine Woche und besonders bevorzugt für einen Monat.

Zusätzlich zu den genannten Vorteilen, die im Wesentlichen auf der Dimensionierung und der Form des erfindungsgemäßen Inhalators beruhen, werden weitere Vorteile gegenüber dem Stand der Technik durch die Merkmale der bevorzugten Ausführungsformen ermöglicht. So gewährleistet der erfindungsgemäße Inhalator eine ausgesprochen einfache und zugleich sichere Anwendung. Darüber hinaus lässt sich der erfindungsgemäße Inhalator auf einfache Weise und kostengünstig herstellen.

Bevorzugte Ausführungsformen des erfindungsgemäßen Inhalators werden nachfolgend mit Verweis auf die folgenden Figuren näher beschrieben. Es zeigen:
- Figuren 1 und 2:: perspektivische Ansichten einer bevorzugten Ausführungsform des erfindungsgemäßen Inhalators;
- Figur 3:: eine perspektivische Schnittansicht des Inhalators der Figur 2;
- Figuren 6 und 7:: Schnittansichten von Details des Inhalators gemäß Figuren 1 und 2;
- Figur 8:: eine Schnittansicht einer bevorzugten Ausführungsform des erfindungsgemäßen Inhalators;
- Figur 9:: eine Schnittansicht einer weiteren bevorzugten Ausführungsform des Inhalators;
- Figur 10:: eine Schnittansicht einer weiteren bevorzugten Ausführungsform des Inhalators;
- Figur 11:: eine Schnittansicht einer weiteren bevorzugten Ausführungsform des Inhalators;
- Figur 12:: eine Schnittansicht einer weiteren bevorzugten Ausführungsform des Inhalators;
- Figuren 13 und 14:: Schnittansichten einer weiteren bevorzugten Ausführungsform des erfindungsgemäßen Inhalators; und
- Figur 15:: eine Schnittansicht einer weiteren bevorzugten Ausführungsform des erfindungsgemäßen Inhalators.

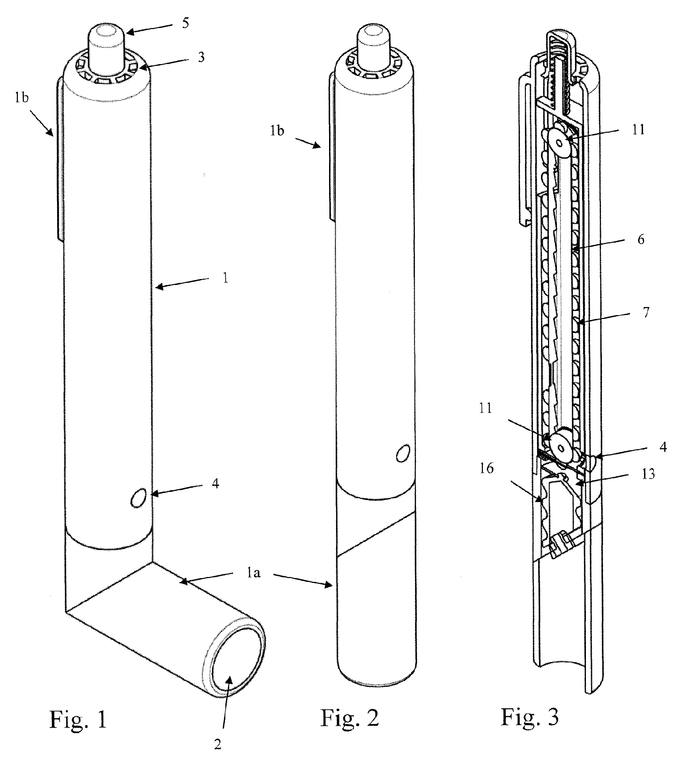

Die Figuren 1-7 zeigen verschiedene Ansichten einer ersten bevorzugten Ausführungsform des erfindungsgemäßen Inhalators. Bei diesem handelt es sich um ein durch Atemluft betätigtes Inhalationsgerät, das eine langgestreckte, annähernd zylindrische Form aufweist. Der Inhalator gemäß der bevorzugten Ausführungsform weist ein im Wesentlichen zylindrisches Gehäuse 1 mit einer Ansaugöffnung 2 und einer oder mehreren Zuluftöffnungen 3 auf. Die Ansaugöffnung 2 ist an einem ersten, unteren Ende 1a des zylindrischen Gehäuses 1 vorgesehen, welches sich aus einer ersten, in Figur 2 dargestellten Position, in der das erste Ende 1a im Wesentlichen parallel zur von etwa 90° einschließt.

In der dargestellten Ausführungsform erfolgt diese Bewegung durch Verdrehen des ersten Endes gegenüber dem restlichen Gehäuse des Inhalators. Im Inneren des Gehäuses 1 befindet sich ein Förderband bzw. Blisterstreifen 6 mit mehreren Wirkstoff- bzw. Medikamentendosiskammern oder -blasen 7. Im Abstand benachbarter Blasen 7 weist der Blisterstreifen sperrklinkenartige Vorsprünge 8 auf, in die ein Zugelement 9 mit Haken 10 eingreifen kann, um den Blisterstreifen 6 im Gehäuse 1 fortzubewegen. Der Blisterstreifen 6 ist hier zu einer endlosen Schlaufe verbunden und wird auf Umlenkrollen 11 geführt, von denen sich je eine im oberen und im unteren Bereich des Gehäuses 1 befindet.

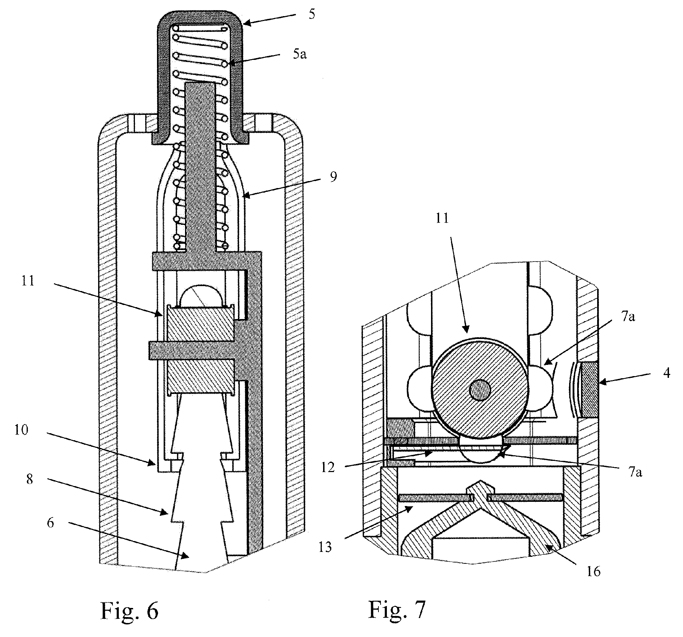

Das Zugelement 9 steht in Verbindung mit einem Druckknopf 5, der von einer Feder 5a in seiner in Figur 6 dargestellten Ausgangsposition gehalten wird. Drückt nun ein Anwender von außen auf den Druckknopf 5, so gleitet das vorzugsweise flexible bzw. biegsame Zugelement 9 mit seinen Haken 10 an den entsprechenden Vorsprüngen 8 der Blisterfolie 6 entlang, bis die Haken 10 in die durch die Vorsprünge 8 gebildeten Einkerbungen eingreifen bzw. einrasten. Wird der Druckknopf 5 vom Anwender freigegeben, so wird durch die integrierte Feder 5a das Zugelement 9 nach oben bewegt, welches mittels der Haken 10 den Blisterstreifen 6 um den Abstand zwischen zwei benachbarten Blasen vorwärts bewegt.

Dabei wird die Blase bzw. der Blister 7 ganz rechts unten auf dem Blisterstreifen aus der Position 7a in die Position 7b (vgl. Figur 7) bewegt, in der die Blase bzw. der Blister 7 durch ein Messer 12 aufgeschnitten wird. Dadurch wird der Blister 7 geöffnet und sein Inhalt fällt auf eine flexible Membran 13, die an einem Ringelement gehalten ist. Die elastische Membran 13 dichtet den Bereich oberhalb der Membran gegen die Gehäusewand nach unten hin ab, so dass die herausgefallene Medikamentendosis nicht über die Ansaugöffnung 2 aus dem Inhalator entweichen kann. Eine entsprechende Membran kann auch weiter oben angeordnet sein, um zu verhindern, dass beim Kippen oder Schütteln des Inhalators das Medikament bzw. der Wirkstoff durch Zuluftöffnungen 3 aus dem Inhalator herausfällt.
durch die Zuluftöffnungen 3 in das Innere des Gehäuses 1 ein. Dadurch kommt es zum Umklappen der Außenkanten der flexiblen Membran 13, so dass die auf der Membran 13 liegenden Medikamentpartikel durch den Luftstrom mitgerissen und in die Atemwege des Anwenders mitgeführt werden. Die lediglich schematisch dargestellte Struktur 16 bildet dabei eine Verwirbelungskammer, die die durchströmende Luft derart verwirbelt, dass der Wirkstoff bzw. das Medikament mit der Luft hinreichend fein vermischt wird.

Das Gehäuse 1 enthält ferner ein Sichtfenster 4, durch das der Anwender den Blister 7 in der Position 7a daraufhin inspizieren kann, ob dieser noch gefüllt ist. Bevorzugt sind die einzelnen Blister mit Nummern versehen, die dem Anwender im Sichtfenster 4 anzeigen, wie viele Dosen des Wirkstoffes noch vorhanden sind. Alternativ kann ein Sichtfenster 4 in Form eines länglichen Schlitzes bereitgestellt werden, mittels dessen sich eine ganze Reihe von Blistern auf Intaktsein überprüfen lassen.

Schließlich enthält der Inhalator gemäß der bevorzugten Ausführungsform einen Clip oder Bügel 1b, der dazu geeignet ist, den Inhalator in einer Hemd- oder Jackentasche zu fixieren.

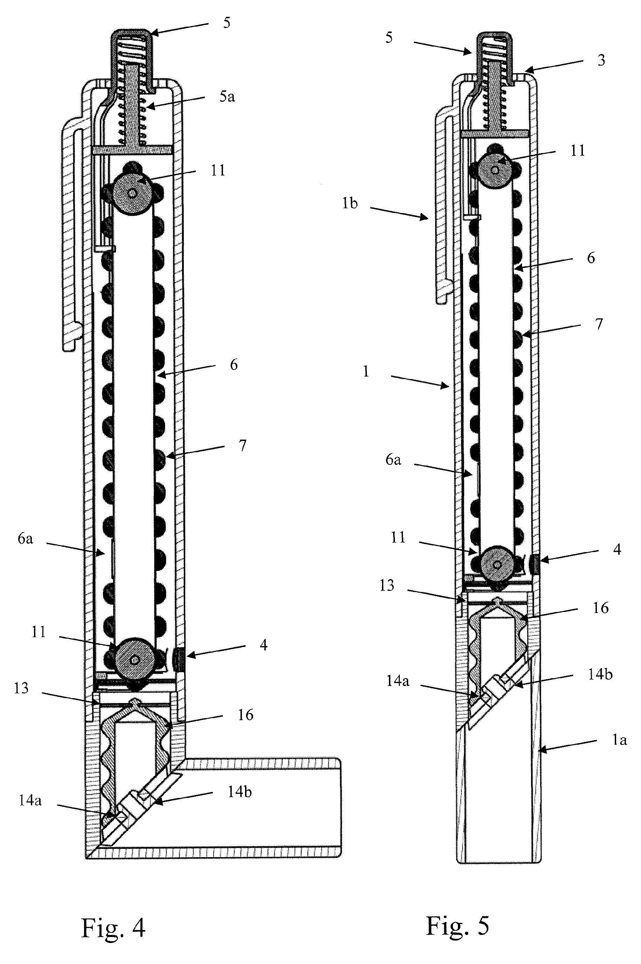

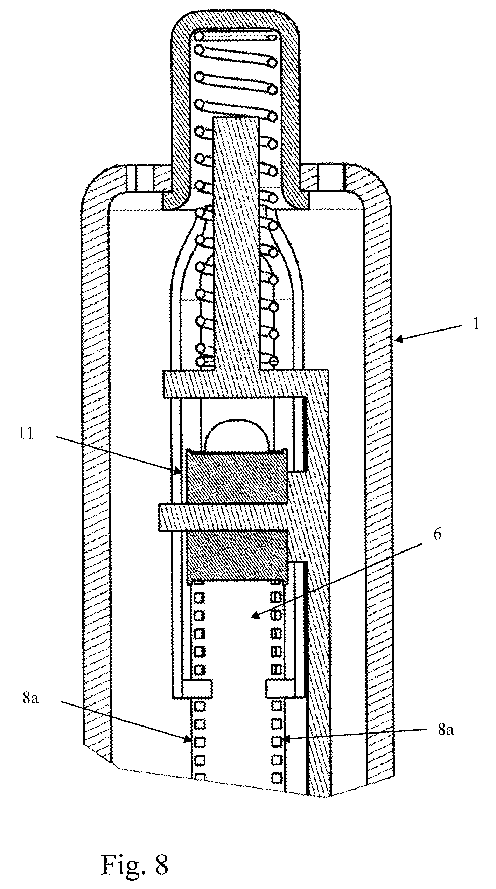

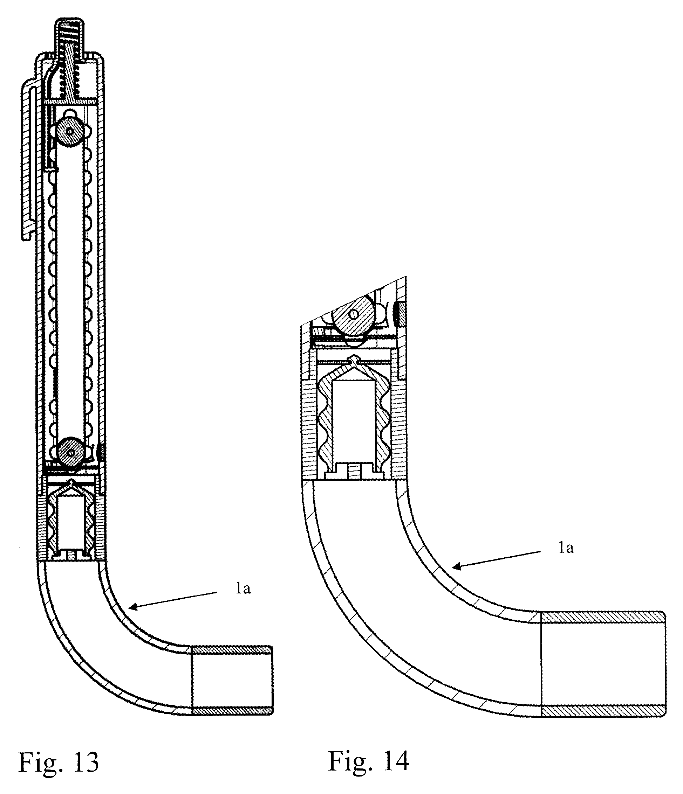

Es versteht sich von selbst, dass die in den Figuren 1-7 dargestellten strukturellen und dimensionalen Details lediglich beispielhaft zu verstehen sind. So ist die Form des Gehäuses 1 nicht auf einen Zylinder eingeschränkt und kann beispielsweise konvex, konkav oder konisch ausgebildet sein. Auch der Querschnitt des Gehäuses 1 kann von der dargestellten Kreisform abweichen und beispielsweise elliptisch, rechteckig, quadratisch, fünfeckig, sechseckig oder vieleckig sein. Auch das Zugelement 9 kann in einer alternativen Ausführungsform anders ausgebildet sein und beispielsweise nur einen Haken 10 auf einer Seite des Förderbandes bzw. Blisterstreifens 6 aufweisen. Entsprechend kann auch der Blisterstreifen dergestalt ausgebildet sein, dass sich lediglich auf einer Seite desselben die dargestellten, sperrklinkenartigen Vorsprünge 8 befinden. Alternativ lässt sich der Blisterstreifen auch mit einer Reihe von Öffnungen 8a ausbilden, wie dies in der Ausführungsform gemäß Figur 8 dargestellt ist, in die Haken oder Spitzen des Zugelements 9 eingreifen können. Diese Öffnungen 8a können zusätzlich oder alternativ auch dazu dienen, den Blisterstreifen bzw. das Förderband auf den In der Ausführungsform gemäß den Figuren 1-7 lässt sich das erste Ende 1a des Inhalators aus der ersten Position in die zweite Position bewegen, indem das erste Ende 1a des Inhalators gegenüber der Längsachse des Inhalators verdreht wird. Hierfür ist der Inhalator, wie aus den Figuren 4 und 5 ersichtlich wird, bevorzugt mit einer Nabe 14a und einer Achse 14b ausgestaltet, die derart ineinander greifen, dass die genannte Drehbewegung ermöglicht wird. Gemäß einer weiteren bevorzugten Ausführungsform, die in den Figuren 13 und 14 dargestellt ist, ist das erste Ende 1a des Inhalators derart flexibel ausgebildet, dass es sich um mindestens 45°, bevorzugt mindestens 60°, stärker bevorzugt mindestens 75° und besonders bevorzugt um etwa 90° gegenüber der Längsachse des Inhalators verbiegen lässt.

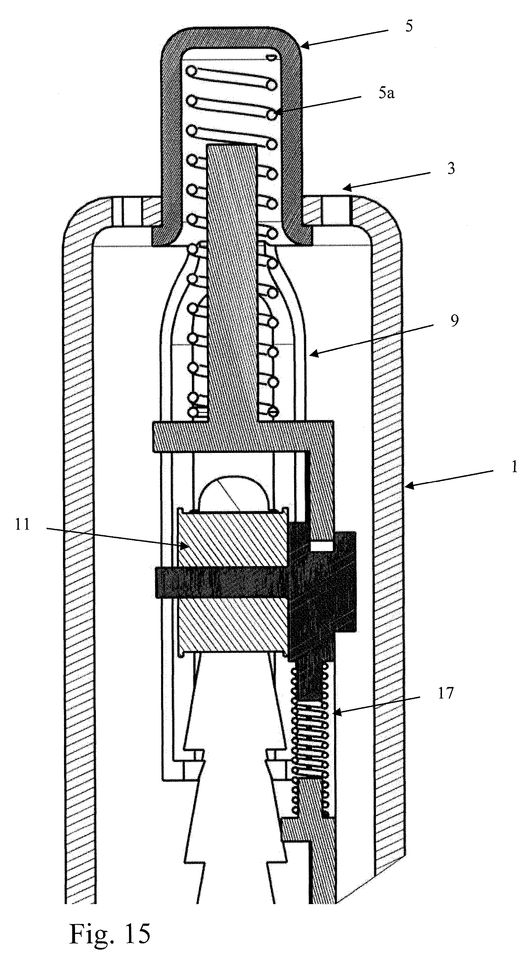

Um den Blisterstreifen 6 mit hinreichend elastischer Spannung auszubilden, dass der Blisterstreifen zwischen den beiden Umlenkrollen 11 gespannt bleibt, ist gemäß der Ausführungsform der Figuren 1-7 der Blisterstreifen 6 mit einem elastischen Element 6a versehen, dass den Blisterstreifen 6 gespannt hält. Gemäß einer weiteren bevorzugten Ausführungsform, die in der Figur 15 zu sehen ist, kann zusätzlich oder alternativ zu dem elastischen Element 6a eine Feder 17 vorgesehen sein, mittels derer eine der beiden Umlenkrollen 11 federelastisch gelagert ist. Es können auch beide Umlenkrollen 11 mittels Federn 17 federelastisch gelagert werden.

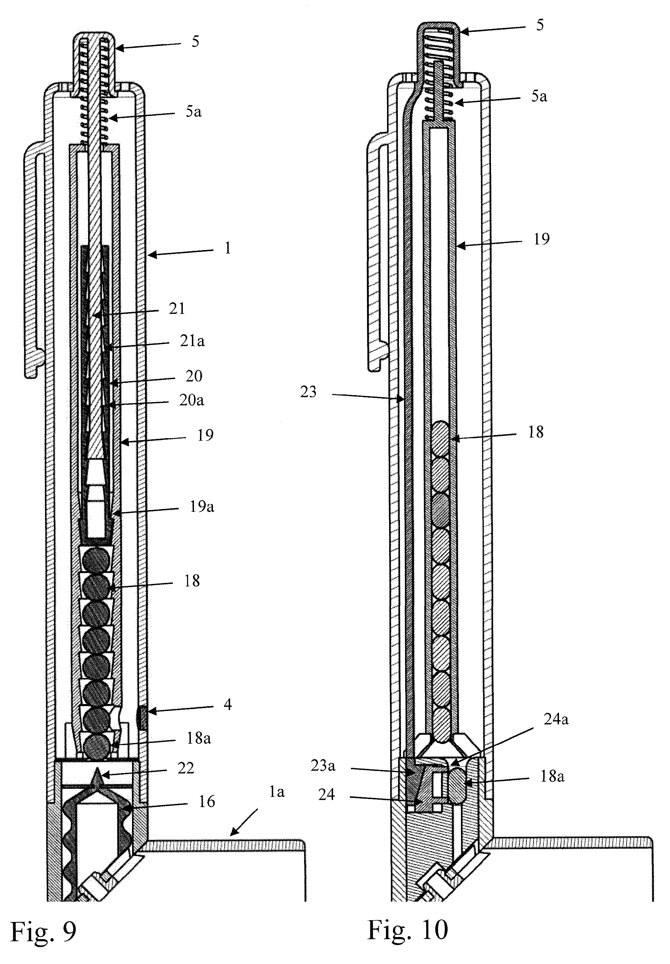

Eine weitere bevorzugte Ausführungsform des Inhalators ist in Figur 9 dargestellt. Anstelle eines Blisterstreifens 6 mit Einzelblistern 7 enthält der Inhalator gemäß dieser Ausführungsform eine Reihe von Wirkstoffkapseln 8, die in einer Röhre 19 übereinander gelagert werden. Innerhalb dieser Röhre 19 befindet sich ein beweglicher Zylinder 20, der mit einem Vorsprung 20a versehen ist, der in die sperrklinkenartigen Vorsprünge 19a innerhalb der Röhre 19 derart eingreift, dass sich der Zylinder 20 innerhalb der Röhre 19 nur abwärts bewegen lässt. Innerhalb des Zylinders 20 ist ein Kolben 21 beweglich gelagert, der an einem Ende ebenfalls Vorsprünge 21 a aufweist, die in sperrklinkenartige Vorsprünge 20b auf der Innenseite des Zylinders 20 derart eingreifen, dass sich der Kolben 21 innerhalb des Zylinders 20 nur nach oben bewegen Zylinder 20 nach unten gedrückt wird, wohingegen beim Loslassen des Druckknopfes 5 nur der Kolben 21 aufgrund der Wirkung der Feder 5a nach oben gezogen wird.

Aufgrund des vom Zylinder 20 auf die Reihe von Kapseln 18 ausgeübten Druckes wird die unterste Kapsel 18a in den Dorn 22 gerammt, wodurch diese geöffnet wird. Mittels eines Sichtfensters 4 lässt sich erkennen, ob sich noch Kapseln 18 innerhalb der Röhre 19 befinden.

Eine weitere bevorzugte Ausführungsform des Inhalators ist in Figur 10 dargestellt. Auch dieser Inhalator weist eine Röhre 19 auf, innerhalb derer eine Reihe von Kapseln 18 gelagert sind. Die unterste Kapsel wird dabei durch die Schwerkraft in die Position 18a geschoben bzw. in dieser gehalten. Der Druckknopf 5 ist bei dieser Ausführungsform über ein Gestänge 23 mit einem keilförmigen Element 23a verbunden. Dieses interagiert mit einem ebenfalls keilförmigen Schneidelement 24, das zwei Dornen oder Messer 24a aufweist. Drückt nun ein Anwender auf den Druckknopf 5, so wird mittels des Gestänges 23 das keilförmige Element 23a nach unten geschoben, wodurch das ebenfalls keilförmige Schneidelement 24 nach rechts bewegt wird, so dass die Kapsel 18a durch die beiden Dornen oder Messer 24a punktiert oder aufgeschnitten wird. Der in der Kapsel enthaltene Wirkstoff kann dann über die Bohrung 25 zur Ansaugöffnung 2 am ersten Ende 1 a des Inhalators entweichen.

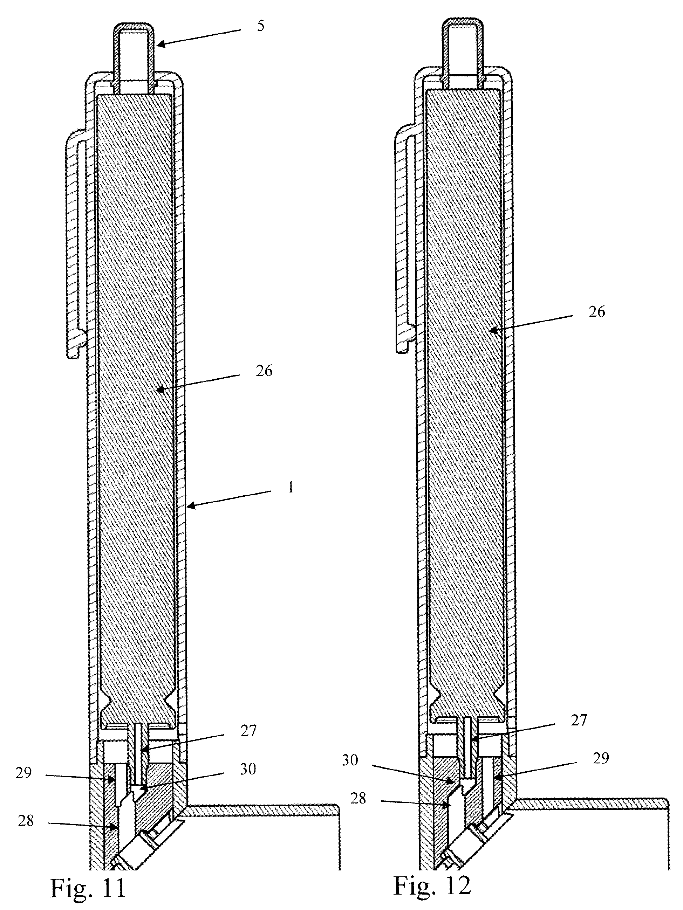

Schließlich sind in den Figuren 11 und 12 zwei weitere bevorzugte Ausführungsformen des Inhalators dargestellt, die auf dem Prinzip von bekannten Flüssigkeitsinhalatoren basieren. Bei diesen Ausführungsformen ist der Wirkstoff in Form einer Flüssigkeit in einem Wirkstoffreservoir 26 vorgesehen. Der Inhalator kann eine Pumpe aufweisen, um eine definierte Flüssigkeitsmenge bereitzustellen oder das Wirkstoffreservoir kann mittels eines Treibmittels unter Druck stehen. Durch Druck auf den Druckknopf 5 wird das Reservoir 26 zusammen mit der Röhre 27 gegen den Anschlag bzw. die Kante 30 gedrückt, so dass ein nicht dargestelltes Ventil geöffnet wird und Flüssigkeit durch die Röhren 27 und 28 austreten kann und so zur Ansaugöffnung 2 gelangt. Bevorzugt ist eine weitere Röhre 29 vorgesehen, die in Kombination mit der Röhre 28 (Figur 11) oder allein (Figur 12) einen Druckausgleich unterhalb des Reservoirs 26 ermöglicht.

## Patentansprüche

1. Inhalator zur wiederholten Verabreichung einer definierten Dosis eines Wirkstoffes in die Atemwege eines Anwenders, wobei der Inhalator eine längliche Form hat, die eine Längsache und eine Querschnittsprofil definiert, wobei der Inhalator mehrere, voneinander separierte Portionen (7; 18) der definierten Dosis des Wirkstoffes enthält, die in Dosisbehältern auf einem Förderband angeordnet sind, **dadurch gekennzeichnet, dass** der Inhalator einen Auslösemechanismus und eine Kanülenspitze oder ein Messer (12) aufweist und wobei ein Betätigen des Auslösemechanismus bewirkt, dass ein Dosisbehälter des Förderbands derart in die Kanülenspitze oder das Messer (12) bewegt wird, dass dieser aufgeschnitten wird wobei die maximale Querschnittsfläche höchstens 4 cm² und die maximale Ausdehnung des Querschnitts höchstens 2,85 cm beträgt.

2. Inhalator nach Anspruch 1, wobei die Portionen in Blasen (7) eines Blisterstreifens (6) angeordnet sind und wobei ein Betätigen des Auslösemechanismus bewirkt, dass eine Blase (7) des Blisterstreifens (6) derart in die Kanülenspitze oder das Messer (12) bewegt wird, dass diese aufgeschnitten wird.

3. Inhalator nach Anspruch 1 oder 2, wobei die maximale Querschnittsfläche höchstens 3 cm², bevorzugt höchstens 2 cm², besonders bevorzugt höchstens 1,5 cm² beträgt.

4. Inhalator nach einem der vorigen Ansprüche, wobei der Inhalator im wesentlichen die Form eines Kugelschreibers aufweist.

5. Inhalator nach einem der vorigen Ansprüche, wobei der Inhalator einen Clip (1b) aufweist, der dazu geeignet ist, den Inhalator in einer Hemd- und/oder Jackentasche zu fixieren.

6. Inhalator nach einem der vorigen Ansprüche, wobei der Inhalator an einem ersten Ende (1a) eine Ansaugöffnung (2) aufweist und wobei das erste Ende (1a) derart beweglich ist, dass es sich aus einer ersten Position, in der das erste Ende (1a) im wesentlichen parallel zur Längsachse des Inhalators ausgerichtet ist, in eine zweite Position bewegen lässt, in der das erste Ende (1a) mit der Längsachse des Inhalators einen Winkel von mindestens 45°, bevorzugt von mindestens 60°, stärker bevorzugt von mindestens 75°, besonders bevorzugt von etwa 90°, einschließt.

7. Inhalator nach einem der vorigen Ansprüche, wobei der Inhalator an einem ersten Ende (1a) eine Ansaugöffnung (2) aufweist und wobei das erste Ende (1a) gegenüber der Längsachse des Inhalators verkippbar und/oder verbiegbar ist.

8. Inhalator nach einem der vorigen Ansprüche, wobei der Inhalator an einem ersten Ende (1a) eine Ansaugöffnung (2) aufweist und wobei das erste Ende (1a) gegenüber der Längsachse des Inhalators verdrehbar ist.

9. Inhalator nach einem der vorigen Ansprüche, wobei der Wirkstoff in Form eines Pulvers vorliegt

10. Inhalator nach einem der Ansprüche 1 bis 8, wobei der Wirkstoff in Form einer Flüssigkeit, bevorzugt in Form einer volatilen Flüssigkeit, vorliegt.

11. Inhalator nach einem der vorigen Ansprüche, wobei der Inhalator eine Mischeinrichtung (16) aufweist, die dazu geeignet ist, einen Luftstrom innerhalb des Inhalators zu verwirbeln.

12. Inhalator nach einem der vorigen Ansprüche, wobei der Inhalator ein Sichtfenster (4) aufweist, durch das die Anzahl der vorhandenen Dosen optisch überprüft werden kann.

13. Inhalator nach einem der vorigen Ansprüche, wobei der Auslösemechanismus einen Druckknopf (5) aufweist und wobei der Druckknopf (5) mittels eines Hakens (10) in sperrklinkenartige Vorsprünge (8) an dem Förderband bzw. Blisterstreifen (6) eingreift.

14. Inhalator nach einem der vorigen Ansprüche, wobei der Inhalator eine flexible Auflagefläche (13) aufweist, die derart angeordnet ist, dass eine Portion der definierten Dosis des Wirkstoffes auf ihr gelagert werden kann.

15. Inhalator nach Anspruch 14, wobei der Inhalator eine Ansaugöffnung (2) aufweist und wobei ein Saugen an der Ansaugöffnung (2) bewirkt, dass die flexible Auflagefläche (13) soweit deformiert wird, dass die darauf gelagerte Portion freigegeben wird.

## Claims

1. An inhaler for repeatedly administering a defined dose of an active ingredient into the respiratory tracts of a user, wherein the inhaler has an elongate shape defining a longitudinal axis and a cross-sectional profile, wherein the inhaler comprises several portions (7; 18) of the defined dose of the active ingredient, wherein said portions (7; 18) are separated from each other and arranged in dose containers on a conveyor belt, **characterized in that** the inhaler comprises a release mechanism and a cannula tip or a knife (12) and wherein the actuation of the release mechanism entails that a dose container of the conveyor belt is moved into the cannula tip or the knife (12) such that it is cut open, wherein the maximum cross-sectional area is at most 4 cm² and the maximum extension of the cross-section is at most 2.85 cm.

2. The inhaler according to claim 1, wherein the portions are arranged in blisters (7) of a blister strip (6) and wherein the actuation of the release mechanism entails that a blister (7) of the blister strip (6) is moved into the cannula tip or the knife (12) such that it is cut open.

3. The inhaler according to claim 1 or 2, wherein the maximum cross-sectional area is at most 3 cm², preferably at most 2 cm², particularly preferably at most 1.5 cm².

4. The inhaler according to any one of the preceding claims, wherein the inhaler has essentially the shape of a ballpoint pen.

5. The inhaler according to any one of the preceding claims, wherein the inhaler comprises a clip (1b) which is adapted for fixing the inhaler in a shirt and/or jacket pocket.

6. The inhaler according to any one of the preceding claims, wherein a first end (1a) of the inhaler comprises a suction orifice (2) and wherein the first end (1a) is movable such that it can be moved from a first position, in which the first end (1a) is aligned substantially parallel to the longitudinal axis of the inhaler, into a second position, in which the first end (1a) and the longitudinal axis of the inhaler include an angle of at least 45°, preferably at least 60°, more preferably at least 75°, particularly preferably about 90°.

7. The inhaler according to any one of the preceding claims, wherein a first end (1a) of the inhaler comprises a suction orifice (2) and wherein the first end (1a) is tiltable and/or bendable relative to the longitudinal axis of the inhaler.

8. The inhaler according to any one of the preceding claims, wherein a first end (1a) of the inhaler comprises a suction orifice (2) and wherein the first end (1a) is rotatable relative to the longitudinal axis of the inhaler.

9. The inhaler according to any one of the preceding claims, wherein the active ingredient is in the form of a powder.

10. The inhaler according to any one of claims 1 to 8, wherein the active ingredient is in the form of a liquid, preferably in the form of a volatile liquid.

11. The inhaler according to any one of the preceding claims, wherein the inhaler comprises a mixing means (16) which is adapted for swirling an air stream within the inhaler.

12. The inhaler according to any one of the preceding claims, wherein the inhaler comprises an inspection window (4) through which the number of the available doses can be optically checked.

13. The inhaler according to any one of the preceding claims, wherein the release mechanism comprises a push-button (5) and wherein said push-button (5) engages by means of a hook (10) with pawl-like projections (8) on the conveyor belt or blister strip (6).

14. The inhaler according to any one of the preceding claims, wherein the inhaler comprises a flexible seat area (13) which is arranged such that a portion of the defined dose of the active ingredient can be placed on it.

15. The inhaler according to claim 14, wherein the inhaler comprises a suction orifice (2) and wherein sucking on the suction orifice (2) entails that the flexible seat area (13) is deformed to such an extent that the portion placed on it is released.

## Revendications

1. Inhalateur pour l'administration répétée d'une dose définie d'une substance active dans les voies respiratoires d'un utilisateur, où ledit inhalateur a une forme oblongue définissant un axe longitudinal et un profil de section transversale, où ledit inhalateur comprend plusieurs portions (7 ; 18) de la dose définie de substance active, séparées les unes des autres, disposées dans des récipients doseurs sur une courroie de transport, **caractérisé en ce que** ledit inhalateur présente un mécanisme de déclenchement et une pointe de canule ou une lame (12), et **en ce qu'**un actionnement du mécanisme de déclenchement entraîne le déplacement d'un récipient doseur de la courroie de transport vers la pointe de canule ou vers la lame (12), de manière à casser celui-ci, la section transversale ayant une surface maximale de 4 cm² et la section transversale une extension maximale de 2,85cm.

2. Inhalateur selon la revendication 1, où les portions sont disposées dans des bulles (7) d'une bande thermoformée (6) et où un actionnement du mécanisme de déclenchement entraîne le déplacement d'une bulle (7) de la bande thermoformée (6) vers la pointe de canule ou vers la lame (12) de manière à ouvrir celle-ci.

3. Inhalateur selon la revendication 1 ou 2, où la section transversale a une surface maximale de 3 cm², avantageusement de 2 cm² et préférentiellement de 1,5 cm².

4. Inhalateur selon l'une des revendications précédentes, où ledit inhalateur a sensiblement la forme d'un stylo.

5. Inhalateur selon l'une des revendications précédentes, où ledit inhalateur comporte une agrafe (1b) permettant de fixer l'inhalateur dans une poche de chemise et/ou de veste.

6. Inhalateur selon l'une des revendications précédentes, où ledit inhalateur présente une ouverture d'aspiration (2) à une première extrémité (1a), et où ladite première extrémité (1a) est mobile de manière à permettre son déplacement d'une première position, où la première extrémité (1a) est sensiblement parallèle à l'axe longitudinal de l'inhalateur, vers une deuxième position, où la première extrémité (1a) forme avec l'axe longitudinal de l'inhalateur un angle d'au moins 45°, avantageusement d'au moins 60°, plus particulièrement d'au moins 75° et préférentiellement de 90° approximativement.

7. Inhalateur selon l'une des revendications précédentes, où ledit inhalateur présente une ouverture d'aspiration (2) à une première extrémité (la), et où la première extrémité (1a) est pivotable et/ou flexible par rapport à l'axe longitudinal de l'inhalateur.

8. Inhalateur selon l'une des revendications précédentes, où ledit inhalateur présente une ouverture d'aspiration (2) à une première extrémité (la), et où la première extrémité (1a) est rotative par rapport à l'axe longitudinal de l'inhalateur.

9. Inhalateur selon l'une des revendications précédentes, où la substance active se présente sous la forme d'une poudre.

10. Inhalateur selon l'une des revendications 1 à 8, où la substance active se présente sous la forme d'un liquide, en particulier sous la forme d'un liquide volatil.

11. Inhalateur selon l'une des revendications précédentes, où ledit inhalateur comprend un dispositif mélangeur (16) prévu pour faire tourbillonner un flux d'air à l'intérieur de l'inhalateur.

12. Inhalateur selon l'une des revendications précédentes, où ledit inhalateur comporte un voyant (4) permettant de contrôler visuellement le nombre de doses présentes.

13. Inhalateur selon l'une des revendications précédentes, où le mécanisme de déclenchement comporte un bouton poussoir (5), et où le bouton poussoir (5) agit au moyen d'un crochet (10) sur des saillies (8) en forme de cliquets sur la courroie de transport ou sur la bande thermoformée (6).

14. Inhalateur selon l'une des revendications précédentes, où ledit inhalateur présente une surface d'appui flexible (13) disposée de telle manière qu'une portion de la dose définie de substance active peut être déposée sur celle-ci.

15. Inhalateur selon la revendication 14, où ledit inhalateur présente une ouverture d'aspiration (2), et où une aspiration sur l'ouverture d'aspiration (2) provoque la déformation de la surface d'appui flexible (13) jusqu'à libération de la portion qui y est déposée.
